# EUROPÄISCHE PATENTANMELDUNG

(11) **EP 4 495 225 A1**
(43) Veröffentlichungstag der Anmeldung: **22.01.2025**
(21) Anmeldenummer: 24187417.1
(22) Anmeldetag: 09.07.2024
(51) Int. Cl.: C12N 1/14, C07D 493/22, C12P 7/66, C12P 17/18, C12R 1/645

(54) **VERFAHREN ZUR BIOTECHNOLOGISCHEN GEWINNUNG DES BLAUGRÜNEN PILZPIGMENTS XYLINDEIN AUS PILZFRUCHTKÖRPERN**

(30) Priorität: 10.07.2023 DE 102023118199
(71) Anmelder: Ahrens-Salzsieder, Dirk Holger, 50354 Hürth (DE)
(72) Erfinder: Ahrens-Salzsieder, Dirk Holger, 50354 Hürth (DE)
(74) Vertreter: dompatent von Kreisler Selting Werner - Partnerschaft von Patent- und Rechtsanwälten mbB

(57) **Zusammenfassung**

Die Erfindung betrifft ein Verfahren zur biotechnologischen Gewinnung des blaugrünen Pilzpigments Xylindein aus den Fruchtkörpern der Askomyzeten *Chlorociboria sp.* Die Erfindung betrifft weiterhin die Verwendung des so gewonnen Xylindeins als Farbstoff für Farben, Lacke, Hautfarbstoffe (Tattoos), als Fluoreszenz-Farbstoff für medizinische Zwecke, als organisatorischer Halbleiter sowie für Vorprodukte dieser Anwendungen.

## Beschreibung

Die vorliegende Erfindung betrifft ein Verfahren zur biotechnologischen Gewinnung des blaugrünen Pilzpigments Xylindein aus den Fruchtkörpern der Askomyzeten *Chlorociboria sp.* Die Erfindung betrifft weiterhin die Verwendung des so gewonnen Xylindeins als Farbstoff für Farben, Lacke, Hautfarbstoffe (Tattoos), als Fluoreszenz-Farbstoff für medizinische Zwecke, als organisatorischer Halbleiter sowie für Vorprodukte dieser Anwendungen.

### Hintergrund der Erfindung

Xylindein ist ein blaugrünes Pigment, das von Pilzen der Gattung *Chlorociboria sp.* produziert wird. Xylindein verbleibt auch nach Absterben des Pilzes im Holz und ist als persistenter Farbstoff zu kategorisieren. Zudem ist Xylindein nicht wasserlöslich, d.h. es wird auch nach längerer Zeit nicht aus dem Holz ausgewaschen. Außerdem gilt es als unempfindlich gegenüber Sonnenlicht und ist somit als chemisch inert zu klassifizieren. Der möglichen Nutzung von Xylindein sind wenig Grenzen gesetzt, da es nur aus organischen unbedenklichen Elementen besteht (C, O und H). Durch die aufwendige Gewinnung sowie die Stabilität des Moleküls steht vor allem die Nutzung als foto-inaktiver Farbstoff im Vordergrund (z.B. für Lacke, Tattoo-Tinte, usw.). Zudem werden dem Farbstoff fluoreszierende Eigenschaften zugeschrieben, was den Einsatz als Fluoreszenz-Farbstoff in der medizinischen Forschung vielversprechend erscheinen lässt. Xylindein stellt chemisch gesehen ein Chinon-Pigment dar. Der chemischen Summenformel: C₃₂H₂₄O₁₀ ist zu entnehmen, dass es sich bei diesem Pigment um ein rein organisches Farbpigment handelt. Dieser Umstand unterscheidet dieses Pigment von vielen heute gebrauchten Farbpigmenten, z.B. von den Azo-Farbstoffen, die durch Schwermetalle verunreinigt und durch viele Stickstoff-Gruppen charakterisiert sind. Die molare Masse von Xylindein beträgt: 568.534 g/mol⁻¹, die CAS-Nr. lautet 3779-11-1, der systematische IUPAC Name ist 8,16-Dihydroxy-3,11-dipropyl-3,4,11,12-tetrahydro-pyrano[4,3-h]pyrano[4',3';5,6]xantheno[2,1,9,8-klmna]xanthene-1,7,9,15-tetraone und es weist die folgende chemische Struktur auf:

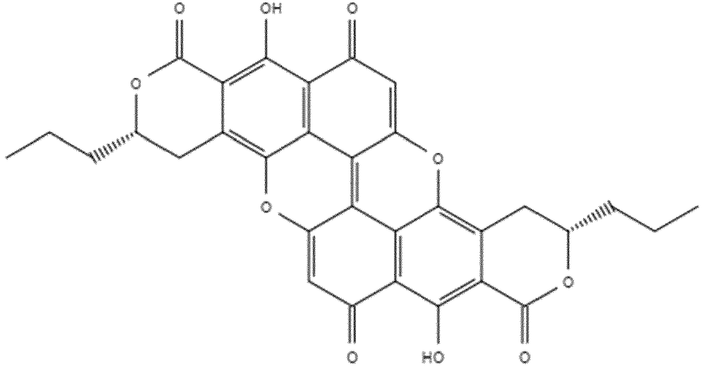

Das Rückgrat von Xylindein bildet das Molekül peri-Xanthenoxanthene (PXX), das als organischer Halbleiter für Transistoren verwendet wird (vgl. R. Harrison et al., Proceedings of SPIE 10101 (2017)). Ein anderer Name für dieses Molekül ist Dinaphthylene dioxide.

Aus der DE 10 2018 127 946 A1 2020.05.14 ist bereits ein Verfahren zur biotechnologischen Gewinnung des blaugrünen Pilzpigments Xylindein in einem Bioreaktor bekannt. Auf den in der DE 10 2018 127 946 A1 gewürdigten relevanten Stand der Technik wird nachfolgend ausdrücklich Bezug genommen.

Bekannt ist dieses Pigment für die natürliche Blaugrünverfärbung von Holz. Dieses im Wald natürlich verfärbte Holz wird seit mehreren Jahrhunderten für Intarsien genutzt. Xylindein ist ebenfalls für die Nutzung als Fluoreszenzmarker oder als organischer Halbleiter vielversprechend. Derzeit gibt es keine Möglichkeit, Xylindein chemisch zu synthetisieren.

Aus der Literatur ist bekannt, Xylindein biotechnologisch herzustellen und aus grünverfärbtem Zellkulturüberstand und grünverfärbter Zellbiomasse zu isolieren. Beispielsweise R. Harrison et al., Proceedings of SPIE 10101 (2017) kultivierten den Pilz *Chlorociboria aeruginosa* zuerst auf Malz-Agarose-Platten, um nach Transfer der Biomasse in einen Bioreaktor im 1L-Maßstab zu kultivieren. S. Stange et al., Holztechnologie 59(1):52-60 (2018) offenbart die Kultivierung der Pilzkultur in Lebensmittelreststoffen wie Orangensaft. Verschiedene Substrate wie Holz, Kaffeesatz, Heu oder Reis wurden getestet. Es wird auch beschrieben, dass nährstofflimitierte Bedingungen in der Form eines niedrigeren Stickstoffgehalts im Vergleich zur verfügbaren Kohlenstoffkonzentration für die Bildung von Xylindein günstig sind. Verschiedene N- und C-Quellen wurden getestet und gemischt. Auch die Heißwasserextraktion von Baumrinden zur Herstellung eines geeigneten Kultivierungsmediums, welche sekundäre Pflanzeninhaltsstoffe beinhalten, wird beschrieben.

Schon S.C. Robinson et al., Color. Technol. 130: 221-225 (2014) wandten das Konzept an, Xylindein direkt aus der Pilzbiomasse und gefärbtem Feststoffsubstrat zu extrahieren, anstatt es aus dem Zellkulturüberstand einer Flüssigkultivierung zu gewinnen. Robinson et al. untersuchten auch die Löslichkeit des Xylindeins in u. a. Acetonitril, Tetrahydrofuran oder Ethanol.

Y. Saikawa et al., Phytochemistry 55:237-240 (2000) extrahierten mittels heißen Chloroforms aus mit *Chlorociboria sp.* befallenem Holz.

G. Weber et al., Color Technol. 130:445-452 (2014) beschrieben die Extraktion von Xylindein mittels Dichlormethan aus einer *Chlorociboria aeruginosa*-Kultur aus Malzagarplatten, bestehend aus 2 % Malz und 1,5 % Agar.

G. Stange et al., Holztechnologie 59(2):47-54 (2018) nutzten dieses Konzept und färbten Vollholz durch Pilzkultivierung in Flüssigmedien wie beispielsweise Orangensaft. Ziel der Arbeit war es, geeignete Holzarten als Substrat für diese Pilzkultivierung zu finden. Sie entwickelten diesbezüglich einen Feststoffreaktor im Prototypenmaßstab zur gezielten mykologischen Holzverfärbung.

Zur Extraktion des Pilzpigments aus verfärbten Substraten wurde in US 2017/0081540 A1 Aceton, Tetrahydrofuran bzw. Acetonitril genutzt. Das Extrakt wurde in einem weiteren Schritt mit einem Öl vermischt, welches nach Evaporation des Lösungsmittels als Flüssigträger für das Pilzpigment fungierte. Somit konnte eine Suspension des Pigments in Öl hergestellt werden. Diese Pigmentsuspension soll als Anstrichstoff eingesetzt werden.

Die Extraktion von Pigmenten mit heißem Wasser, sowohl aus befallenem Holz als auch aus Pilzmyzel, wird in EP 1 736 053 A1 nahegelegt. Als Reinigungsmethode wird das Ausfällen durch Ansäuern in Kombination mit Filtration beschrieben. Zusammenfassend geht aus dem Stand der Technik hervor, dass eine Kultivierung bisher nur im 1L-Maßstab realisiert werden kann. Die beschriebenen Kultivierungszeiten sind sehr lang. Sie betragen mindestens 6 Wochen. Die bei der Extraktion eingesetzten Lösungsmittel sind für die industrielle Anwendung aufgrund hoher Toxizität und der damit verbundenen notwendigen Sicherheitsvorkehrungen nicht praktikabel.

Darauf aufbauend beschreibt die DE 10 2018 127 946 A1 bzw. die Nachanmeldung WO 2020/094552 ein Verfahren zur biotechnologischen Gewinnung des blaugrünen Pilzpigments Xylindein in einem Bioreaktor, mit den Schritten:
(a) Kontaktieren des Reaktorinhalts mit Biomasse der Pilzkultur *Chlorociboria sp.* und Rühren des Reaktorinhalts unter Bildung von Xylindein-haltiger Biomasse,
(b) Abtrennung der Xylindein-haltigen Biomasse, die sich in Schritt (a) gebildet hat,
(c) Extraktion des Xylindeins aus der in Schritt (b) abgetrennten Biomasse mit einem Lösungsmittel, wobei die Biomasse, mit der in Schritt (a) kontaktiert wird, ungefärbte Biomasse ist.

Zum Einsatz kommt bei dem Verfahren gemäß DE 10 2018 127 946 A1 ein Bioreaktor bis zu einer Größe von 70 I. Das genannte Verfahren soll eine überraschend kurze Kultivierungsdauer erlauben (was zu wesentlich kürzeren Prozesszeiten führt, und damit mehr Xylindein pro Zeit als mit herkömmlichen Verfahren gewonnen werden kann) und soll angeblich holzfrei erfolgen können (was aber nur in einer speziellen Ausführungsform gewährleistet ist, denn nach Absatz 0020 kann auch mit Pilzkultur befallenes Holz als Substrat zur Anwendung kommen). Der Vorteil der holzfreien Verfahrensführung liegt darin begründet, dass zum einen das Wachstum einer Pilzkultur auf Holz deutlich langsamer zu klassifizieren ist als auf anderen organischen Substraten (wenig Stickstoff). Dies ist zum einen bedingt durch das generell langsame Wachstum des Organismus und zum anderen durch die Festigkeit bzw. chemische Komposition von Holz. Da Holz ein heterogener "Verbundstoff" aus Lignin, Cellulose und Hemicellulose ist, können andererseits einzelne Bestandteile des Holzes (z.B. Phenole) bei der Extraktion des Xylindeins von Nachteil sein und bedürfen Lösungsmittel mit bedenklicher Toxizität.

Die Nachteile der biotechnologischen Verfahren mittels Einsatzes von Bioreaktoren wie in der DE 10 2018 127 946 A1 sind deren hohe technische Komplexität, der damit verbundene hohe technische Aufwand und daraus resultierend, hohe Kosten für die Herstellung des Xylindeins.

Aufgabe der vorliegenden Erfindung ist daher die Bereitstellung eines Verfahrens zur Herstellung von Xylindein mit möglichst geringer technischer Komplexität, um den technischen Aufwand und die erforderlichen Herstellungskosten gering zu halten, und dass möglichst toxikologisch unbedenkliche Lösungsmittel bei der Isolierung eingesetzt werden können.

### Kurzbeschreibung der Erfindung

Ausgehend von den in NRW recht häufig vorkommenden Pilzen der Gattung *Chlorociboria sp.* wurde gefunden, dass durch Isolation aus den Fruchtkörpern der Pilze, die aus natürlichen Quellen bzw. aus Kultur auf einem holzhaltigem Nährmedium erhältlich sind, das Xylindein durch einfache und nachhaltige Reinigung in hoher Reinheit isoliert werden kann. Erfindungsgemäß wird die gestellte Aufgabe durch ein Verfahren mit den Merkmalen der unabhängigen Patentansprüche 1 und 11 gelöst. In einem ersten Aspekt betrifft die vorliegende Erfindung ein Verfahren zur biotechnologischen Gewinnung des blaugrünen Pilzpigments Xylindein aus Fruchtkörpern der Pilzkultur *Chlorociboria sp.,* umfassend die folgenden Schritte:
(a) Aufbringen Kultivieren und zum Fruchten bringen einer *Chlorociboria sp.* Pilzkultur auf einem Nährmedium umfassend Naturholz oder Mischungen von verschiedenen Naturhölzern;
(b) Ernten der Fruchtkörper von der gemäß Schritt (a) kultivierten Pilzkultur;
(c) Einbringen der in Schritt (b) geernteten Fruchtkörper in ein Lösungsmittel oder Lösungsmittelgemisch und
(d) Extrahieren des Xylindeins aus den geernteten Fruchtkörpern mit dem Lösungsmittel oder Lösungsmittelgemisch.

Das Verfahren des ersten Aspekts kann dabei weiterhin einen oder mehrere der folgenden Schritte umfassen:
(a₀) Vorkultivieren einer isolierten *Chlorociboria sp.* Pilzkultur oder Sporen derselben auf einem Agar-Nährmedium;
(e) Abtrennen des Xylindeins von dem Lösungsmittel oder dem Lösungsmittelgemisch;
(f) Trocknen des Xylindeins; und
(g) Reinigen des in Schritt (e) oder (f) erhaltenen Xylindeins

In einem zweiten Aspekt betrifft die vorliegende Erfindung ein Verfahren zur biotechnologischen Gewinnung des blaugrünen Pilzpigments Xylindein aus Pilzfruchtkörpern von *Chlorociboria sp.,* umfassend Ernten von natürlich vorkommenden Fruchtkörpern von *Chlorociboria sp.* und Isolieren des Xylindeins daraus nach Schritt (c) und fortfolgenden Schritten gemäß dem vorstehend genannten und nachfolgend weiter beschriebenen ersten Aspekt der Erfindung.

In einem dritten Aspekt betrifft die vorliegende Erfindung die Verwendung des nach dem ersten oder zweiten Aspekt gewonnen Xylindeins, das - bedingt durch seine Reinheit - direkt als Farbstoff für Farben, Lacke, Hautfarbstoffe (Tattoos), als Fluoreszenz-Farbstoff für medizinische Zwecke, als organisatorischer Halbleiter sowie für Vorprodukte dieser Anwendungen eingesetzt werden kann.

### Kurzbeschreibung der Figuren

Figur 1 zeigt eine blau-grünes Myzel des Pilzes *Chlorociboria sp.* in Petrischale auf Agarplatte von Beispiel 1.
Figur 2 zeigt einen in Beispiel 1 eingesetzten Spritzenvorsatzfilter.
Figur 3 zeigt einen blau-grünes Myzel des Pilzes *Chlorociboria sp.,* aufgebracht auf Holzstücke im Glasgefäß gemäß Schritt (a) von Beispiel 1.
Figur 4 zeigt Fruchtkörper auf Holz gemäß Schritt (b) von Beispiel 1
Figur 5 zeigt Fruchtkörper auf Holz gemäß Schritt (b) von Beispiel 1, vergrößert
Figur 6 zeigt Fruchtkörper in Aceton gemäß Schritt (d) von Beispiel 1.
Figur 7 zeigt blau-grünes Myzel des Pilzes *Chlorociboria sp.,* aufgebracht auf gehäckseltem Holzgemisch gemäß Schritt (a) von Beispiel 3.
Figur 8 zeigt kristalline Xylindeinpigmente am äußeren Rand der Schale, die gemäß der Aufreinigung von Beispiel 4 erhalten wurden.

### Ausführliche Beschreibung der Erfindung

In dem Verfahren des ersten Aspekts der Erfindung werden in Schritt (a) als Nährmedium umfassend Naturholz oder Mischungen von verschiedenen Naturhölzern vorzugsweise solche Nährmedien eingesetzt, die im Wesentlichen neben dem für die Kultivierung und zum Fruchten bringen erforderlichen Menge an Wasser aus dem Naturholz oder den Mischungen aus verschiedenen Naturhölzern bestehen. Dabei können das/die eingesetzte(n) Naturhölzer vollständig sterilisierte, teilweise sterilisierte oder unbehandelte Naturhölzer sein. Es können dabei Holzspäne, Holzdübel und/oder größere Holzstücke des Naturholzes bzw. der Naturhölzer eingesetzt werden. Dabei sind Weichhölzer wie Pappel, Weide und Birke besonders bevorzugt. Die Nährmedien sind mit Wasser ständig feucht zu halten. Eine regelmäßige Sichtkontrolle ist erforderlich, ggfls. ist Wasser zuzuführen. Das Nährmedium kann dabei zusätzlich geeignete Supplemente, organische oder anorganische Supplemente/Zusatzstoffe, insbesondere organischer Stickstoffverbindungen/Stickstoffquellen (wie NH₄⁺-haltige Verbindungen, einschließlich Aminosäuren, andere organische Ammoniumverbindungen und anorganische Ammoniumsalze, stickstoffhaltige Polymere, sowie Nitrat- und Nitrit-haltige Verbindungen) enthalten. Chlorociboria kann potentiell alle Derivate von Stickstoff verwerten mit Ausnahme von molekularem Stickstoff. Die erforderlichen Supplemente liegen dabei vorzugsweise in der wässrigen Komponente des Nährmediums vor.

Dabei ist Nährmedium aus sterilisiertem Naturholz oder Mischungen desselben besonders bevorzugt, da diese erfindungsgemäß zu höheren Ausbeuten Xylindeins führt.

Die erforderliche Sterilisation des Holzes kann dabei z.B. im Autoklav, mittels ionisierender Strahlung o.ä. erfolgen. Die nachfolgenden Schritte dieses Verfahrens können wie folgt erfolgen:
- Aufbringen (Inokulation) der Pilzkultur auf sterilisiertem Naturholz oder in geeignete Mischungen aus Naturholz mit Supplementen;
- zum Fruchten bringen der Pilzkultur;
- Ernten der Fruchtkörper;
- Einbringung der Fruchtkörper in ein Lösungsmittel;
- Extraktion von Xylindein in Lösungsmittel;
- Abtrennung des Lösungsmittels und
- Trocknen der Xylindeinpartikel.

Bevorzugt erfolgen die genannten Schritte nacheinander in genau dieser Reihenfolge.

Das Verfahren zur Kultivierung der Pilzkultur *Chlorociboria sp.* auf Holz als Substrat ist bereits seit längerer Zeit bekannt. Der Vorteil des erfindungsgemäßen Verfahrens ist die Gewinnung des blaugrünen Pilzpigments Xylindein aus Fruchtkörpern der Pilzkultur *Chlorociboria sp.* Dazu wird die Pilzkultur *Chlorociboria sp.* zunächst auf Naturholz oder auf geeignete Mischungen aus Naturholz, das/die optional mit geeigneten Supplementen versetzt sind, aufgebracht.

Bei den Holzsorten hat sich gezeigt, dass Weichhölzer wie Pappel, Weide und, Birke eigentlich Harthölzern wie Eiche, Esche, Buche vorzuziehen sind, es wurde jedoch auch gefunden, dass der Pilz auch sehr gut auf Buche wächst.

Holz stellt ein kostengünstiges Substrat dar. Aufwendige technische Einrichtungen und Anlagen, wie z.B. der technisch und kostenmäßig aufwendige Betrieb von Bioreaktoren bzw. Flüssigkulturen entfallen. Die Supplementierung mit Stickstoff bzw. Salzen oder Estern der Salpetersäure können optional die Wachstumsgeschwindigkeit erhöhen.

Die Supplementierung mit zusätzlichen organischen oder anorganischen Zusatzstoffen, z.B. mit Stickstoff in Form organischer Verbindungen (z.B. NH₄⁺, d.h., Ammoniumverbindungen) können ebenfalls die Wachstumsgeschwindigkeit erhöhen.

Die Inokulation mit Pilz-Myzel erfolgt entweder mit geschnittenen Stücken aus bewachsenen Agar-Kulturen oder mittels Homogenisierung der Agar-Kulturen mit sterilisiertem Wasser in einem Mixer und anschließender Aufbringung. Letzteres Verfahren hat den Vorteil, dass mehrere kleinere Myzel-Stücke für eine bessere Verteilung bei der Inokulation und schnelleres Durchwachsen des Substrats sorgen.

Das Naturholz oder die geeigneten Mischungen aus Naturholz mit Supplementen mit der aufgebrachten (angeimpften) Pilzkultur *Chlorociboria sp.* werden zunächst in geeigneten Behältnissen (z.B. Beuteln, Gläsern, Flaschen, jeweils mit Filtern) bei Raumtemperatur gelagert und dabei von *Chlorociboria sp.* durchwachsen. Die *Chlorociboria sp.* Kulturen wachsen unter partiell anaeroben Bedingungen und müssen nicht künstlich belichtet werden. In den Gefäßen wird sich durch den Wachstumsprozess der Pilzkulturen der Sauerstoffgehalt reduzieren, während sich der CO₂-Anteil erhöhen wird. Je nach Form der Gefäße werden sich ausgeprägte CO₂-Senken bilden. Bedingt durch die eingesetzten Filter wird der Sauerstoffanteil allerdings nicht gegen Null tendieren. Vollständig anaerobe Zustände werden somit in den Gefäßen nicht eintreten. Die Luftfeuchtigkeit in den Gefäßen tendiert in Richtung Maximalwert, auch weil zur Erhaltung der Feuchtigkeit des Nährmediums gegebenenfalls Wasser zugeführt werden muss.

Generell herrschen bei Chlorociboria wie bei Pilzen allgemein in ihrer Vegetationsphase eher partiell anaerobe Bedingungen in ihrer Lebensumgebung (Pilze im Boden, Pilze im Holz). Nach derzeitigem Kenntnisstand scheint es als plausibel, dass Pilze entgegen früheren gegensätzlichen Annahmen während ihrer Wachstumsphase weniger Sauerstoff benötigen, um zu wachsen. Die genauen Werte (CO₂ - O₂ Gehalt) divergieren von Pilzart zu Pilzart und wurden empirisch bisher nicht festgelegt. Im Grunde lässt sich zusammenfassen: der genaue CO₂- oder O₂-Gehalt ist nachrangig, da alle Pilze bis auf obligat anaerobe Pilze bei jeglichen O₂- bzw. CO₂- Gehalten wachsen und es auch innerhalb einer Art oder Spezies Unterschiede zwischen einzelnen Stämmen geben könnte.

Zur Fruchtung muss generell der Gehalt an O₂ angehoben werden. Es ist aber auch möglich, vereinzelte Fruchtungen in CO₂ armen Umfeldern wie den zur Kultivierung verwendeten Flaschen zu beobachten. Das bedeutet: auch der O₂- Gehalt, der zur Fruchtung notwendig ist, unterscheidet sich von Pilzart zu Pilzart. Auch innerhalb einer Art oder Spezies können unterschiedliche Pilzstämme zu unterschiedlichen O₂-Gehalten zu Fruchten beginnen. Generell sollte der O₂-Gehalt möglichst hoch liegen, also der normalen Atmosphäre entsprechen. Allerdings liegt dieser im Zielkonflikt mit der Luftfeuchte. So geben vielmehr die verwendeten Geräte die klimatischen Bedingungen vor. Die Belüftung muss demnach mit möglichst hoher Luftfeuchte realisiert werden, z.B. Ultraschallverdampfer, der Wasser vor den Lüfter der Zuluft verdampft. Die entstehenden klimatischen Bedingungen werden somit (a) vom verdampften Wasser und (b) von der Menge an Luft bestimmt, die so erzeugt wird, also der Lüftergeschwindigkeit.

Eine Fruchtung kann aber auch schon in einer Tupperdose, mit von Wasser halb bedecktem inokulierten Holz ohne aktive Belüftung, aber mit Belüftungsloch erfolgen. Das heißt die Bedingungen können äußerst variabel sein.

Die Pilzkultur *Chlorociboria sp.* wird bevorzugt in einer Klima-Kammer zum Fruchten gebracht, da diese eine Steuerung von Luftfeuchte; CO₂-Gehalt & künstliche Bewässerung; optional steril/unsteril ermöglicht. Die Temperatur sollte sich an der normalen Raumtemperatur orientieren, die Luftfeuchtigkeit kann in Richtung Maximalwert tendieren und der CO₂-Anteil sollte je nach Wachstumsprozess etwas über das normale Niveau erhöht werden. Die Pilzkultur kann aber, alternativ zu einer Klimakammer, auch in Gefäßen oder geeigneten anderen Räumen zum Fruchten gebracht werden, insbesondere weil bei einigen Stämmen auch ein spontanes Fruchten ohne Klimakammer erfolgen kann. Diese Stämme sind zu bevorzugen, um sich den Aufwand für die Klimakammer zu sparen. Hier gelten grundsätzlich die gleichen klimatischen Bedingungen wie bei der Kultivierung.

Das Ernten der Fruchtkörper der Pilzkultur *Chlorociboria sp.* erfolgt in Form des Absammelns, z.B. mittels Skalpell oder Pinzette. Die Fruchtkörper reißen bzw. brechen an ihrer Basis ab. Holzbestandteile werden nicht mit eingesammelt.

Die so geernteten Fruchtkörper werden nachfolgend in Schritt (c) in ein Lösungsmittel oder Lösungsmittelgemisch eingebracht, wobei dies so zu verstehen ist, dass ausschließlich geerntete Fruchtkörper, die frei von anderen Bestandteilen des *Chlorociboria sp.* sind, gelöst und nachfolgend in Schritt (d) extrahiert werden. Bei diesem erfindungsgemäßen Verfahren wird die Pilzkultur *Chlorociboria sp.* auf Holz - ggfls. mit Supplementen - kultiviert und zum Fruchten gebracht. Die Fruchtkörper selbst bleiben aber holzfrei.

In einer Ausführungsform werden die geernteten Fruchtkörper der Pilzkultur *Chlorociboria sp.* zunächst getrocknet, dann in ein Lösungsmittel eingebracht und so das Pilzpigment Xylindein extrahiert.

In einer weiteren Ausführungsform werden die geernteten Fruchtkörper der Pilzkultur *Chlorociboria sp.* zunächst getrocknet, dann zerkleinert (z.B. durch Mahlen), dann in ein Lösungsmittel eingebracht und so das Pilzpigment Xylindein extrahiert.

In einer weiteren Ausführungsform werden die geernteten Fruchtkörper der Pilzkultur *Chlorociboria sp.* zunächst zerkleinert (z.B. durch Mahlen), dann getrocknet, dann in ein Lösungsmittel eingebracht und so das Pilzpigment Xylindein extrahiert.

In einer weiteren Ausführungsform werden die geernteten Fruchtkörper zunächst zerkleinert (z.B. durch Mahlen), dann in ein Lösungsmittel eingebracht und so das Pilzpigment Xylindein extrahiert (Flüssig-Flüssig-Extraktion).

Die Gewinnung des Xylindeins in den beschriebenen Ausführungsformen ist abhängig von den verwandten Lösungsmitteln.

In der Literatur wird der Einsatz vor allem folgender Lösungsmittel beschrieben: Dichlormethan, Ethanol, heißes Chloroform, Aceton, Acetonitril, Tetrahydrofuran, die zuletzt genannten 3 mit Öl vermischt, 2-Butanon, eine Mischung aus Aceton und 2-Butanon.

Je nach verwandtem Lösungsmittel sind unterschiedliche Wege erforderlich, um die Xylindeinpigmente zu generieren.

Dichlormethan z.B. verflüchtigt sich (Evaporation) und lässt die Pigmente zurück. Beim Einsatz von Aceton ist diesem Wasser hinzuzugeben, damit das Xylindein ausfällt. Das Wasser-Aceton-Gemisch ist dann abzuschöpfen, zu zentrifugieren oder es ist zu (ultra-)filtrieren, um die Xylindeinpigmente zu generieren.

Die gewonnenen Xylindeinpigmente sind anschließend, je nach gewünschtem Gehalt an Restfeuchtigkeit, zu trocknen (Länge/Zeit und Temperatur).

Die zu verwendende Technik (z.B. Rotationsverdampfer) ist abhängig vom eingesetzten Lösungsmittel und deren unterschiedliche Verdampfungs- bzw. Siedepunkten.

Es kann angenommen werden, dass mit der Verwendung der Sporen der Pilzkultur *Chlorociboria sp.* ebenso verfahren werden kann, wie in den vorstehend beschriebenen Schritten beschrieben, um äquivalente Ergebnisse zu erzielen.

In einer Ausführungsform des erfindungsgemäßen Verfahrens ist die Pilzkultur *Chlorociboria sp.* ausgewählt aus *Chlorociboria aeruginascens* und *Chlorociboria aeruginosa.*

Ein weiterer Vorteil des diesem erfindungsgemäßen Verfahren zu Grunde liegenden Verfahrens ist die Reinheit des aus der Extraktion aus Pilzfruchtkörpern gewonnen Xylindeins, da keine Holzbestandteile und Medienbestandteile wie Zucker, Fette oder Aminosäuren mit extrahiert werden. Bedingt durch diese hohe Reinheit des gewonnenen Xylindeins und der verwendeten physiologisch unbedenklichen Extraktions- und Lösungsmittel kann das mit dem erfindungsgemäßen Verfahren erhaltene Xylindein direkt und ohne weitere Reinigungsschritte gemäß dem dritten Aspekt der Erfindung weiterverwendet werden. Eine weitere Reinigung des erhaltenen Xylindeins ist daher nur dann erforderlich, wenn für die Verwendung des Xylindeins besondere Reinheitsanforderungen gestellt werden. Dies kann dabei durch Umkristallisation und/oder Ausfällen erfolgen. Solch eine Kristallisation von Xylindein aus Phenol wurde bereits von C. Liebermann, Berichte der Deutschen Chemischen Gesellschaft zu Berlin 7(2):1102-1103 (1874) beschrieben. Da eine Kristallisation mit der toxischen Verbindung Phenol bedenklich ist, können alternativ hierzu auch die bei der Isolierung in Schritten (c) und (d) verwendeten Lösungsmittelgemische zur Umkristallisation/Ausfällung verwendet werden.

Ein weiterer Vorteil des erfindungsgemäßen Verfahrens ist die Lagerfähigkeit und auch die Transportfähigkeit des Zwischenprodukts "Fruchtkörper" sowie der getrockneten Xylindeinpigmente. Einzelne Verfahrensschritte können daher an verschiedenen Orten durchgeführt werden.

Weitere Vorteile dieses erfindungsgemäßen Verfahrens sind der geringe technische Aufwand und daraus resultierend die geringen Produktionskosten. Es entstehen de facto vor allem Kosten für die Beschaffung und die Lagerung des Holzes.

Ein weiterer Vorteil des erfindungsgemäßen Verfahrens ist dessen ökologische Nachhaltigkeit, insbesondere gegenüber komplexen Kultvierungsverfahren wie in der DE 10 2018 127 946 A1 beschrieben.

Die in dem erfindungsgemäßen Verfahren eingesetzten Kulturen der Gattung Chlorociboria sind dazu befähigt sich vegetativ zu vermehren (Myzelwachstum), zudem können Athrosporen/Konidiosporen durch das Pilzmyzel gebildet werden (= nicht geschlechtliche Fortpflanzung). Die nicht geschlechtlichen Sporen, quasi eine Nebenfruchtform, führen wahrscheinlich zur Bildung von imperfekten Chlorociboria-Formen, die wiederum der Adaption und Seneszenz unterworfen sind. Das erfindungsgemäße Verfahren sieht die geschlechtliche Fortpflanzung des Pilzes vor, d.h. die Pilze bilden Fruchtkörper der Hauptfruchtform und auf diesen Fruchtkörpern geschlechtliche Sporen. Dadurch wird eine Adaption an die Kulturbedingungen und in der Folge eine Seneszenz des Pilzstammes verhindert, anders als bei Verfahren, die nicht zur Bildung der Hauptfruchtform führen, da diese in unregelmäßigen Abständen darauf angewiesen sind, neue Pilzstämme aus der Umwelt zu isolieren, da sie ihre Fähigkeiten verlieren. Die vereinfachte Adaption an die jeweiligen Kultivierungsbedingungen stellt einen weiteren Vorteil des erfindungsgemäßen Verfahrens dar.

In einer bevorzugten Ausführungsform des erfindungsgemäßen Verfahrens ist dem Verfahren mindestens ein Vorkultivierungsschritt auf/in einem geeigneten Nährmedium (ao) vorgeschaltet. Solch eine Vorkultur kann auf folgenden Nährmedien erfolgen: Malz Agar (10 g/l Malz pro 11 Agar-agar g/l); Vorkultur auf YPD-Medium (Glucose, Malz, Hefeextrakt, Pepton); Apfelschalenagar und Kartoffelagar.

Das mit Pilzmyzel durchwachsene Nährmedium wird mittels Mixen/Zerkleinerung mit einem Stabmixer unter Zugabe von sterilisiertem Wasser homogenisiert. Allerdings ist normales Leitungswasser gegenüber aqua dest. auf Grund eines möglichen osmotischen Schocks der Zellen zu bevorzugen.

Das erfindungsmäße Verfahren des zweiten Aspekts umfasst auch die Gewinnung von Xylindein aus Fruchtkörpern der Pilzkultur *Chlorociboria sp.,* die nicht aus dem beschriebenen Kultivierungsverfahren gewonnen wurden (z.B. gesammelte aus dem Wald). Das Isolieren des Xylindeins daraus nach Schritt (c) und fortfolgenden Schritten erfolgt analog den vergleichbaren Schritten des Verfahrens des ersten Aspekts der Erfindung. Die bevorzugten Ausführungsformen dieser Schritte wurden vorstehend ausführlich beschrieben, auf diese wird hier explizit Bezug genommen. So werde auch hier ausschließlich geerntete Fruchtkörper, die frei von anderen Bestandteilen des *Chlorociboria sp.* sind, gelöst und nachfolgend in Schritt (d) extrahiert.

Die Verwendung des nach dem ersten oder zweiten Aspekt gewonnen Xylindeins als Farbstoff für Farben, Lacke, Hautfarbstoffe (Tattoos), als Fluoreszenz-Farbstoff für medizinische Zwecke, als organisatorischer Halbleiter, sowie für Vorprodukte dieser Anwendungen gemäß dem dritten Aspekt der Erfindung kann - bedingt durch seine Reinheit - ohne zusätzliche Aufreinigung erfolgen. Das isolierte Xylindein kann somit unmittelbar in einer für die entsprechende Anwendung geeigneten Trägersubstanz oder Lösungsmittel gelöst und direkt, nach Zugabe erforderlicher Hilfsstoffe, eingesetzt werden.

Die Erfindung wird anhand der nachfolgenden Beispiele näher erläutert. Diese Beispiele sind jedoch nicht als Einschränkung der Erfindung zu betrachten.

### Beispiele

### Beispiel 1

### Vorkultivierung des Xylindein produzierenden Pilzes Chlorociboria so.

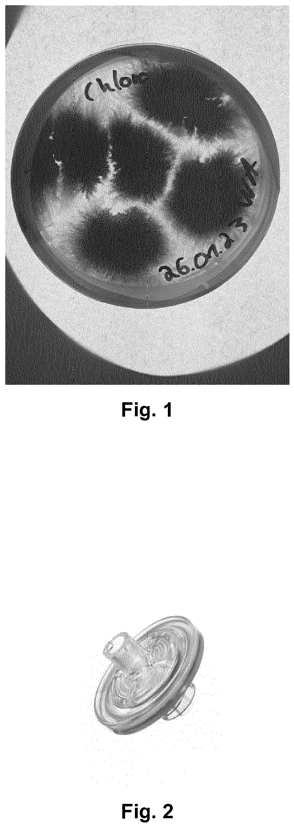

Der isolierte Pilz *Chlorociboria sp.* wurde in einer Petrischale vorkultiviert. Als Nährmedium wurde Malz Agar (10 g/l Malz pro 11 Agar-agar g/l) eingesetzt. Die Petrischale und das Nährmedium wurden zunächst bei 121 °C für 20 min autoklaviert. Die Agar-Platten wurden mit Impfstücken (Plaque, 1-2 cm²) beimpft und über einen Zeitraum von 4-12 Wochen bei etwa 20-22 °C inkubiert. In dieser Zeit zeigte sich blau-grünes Pilzmyzel auf den Agar-Platten. Solch eine blau-grünes Myzel des Pilzes *Chlorociboria sp*. in Petrischale auf Agarplatte ist in Figur 1 gezeigt.

### Schritt (a): Aufbringen und Kultivieren einer Chlorocibora sp. Pilzkultur auf sterilisiertem Naturholz

Zunächst wurden Holzstücke in einer Größe von Holzdübeln (Länge von etwa 3-8 cm und Durchmesser von etwa 0,5 cm) für einen Tag in Wasser eingelegt. Dann wurde das Wasser bis auf einen kleinen Rest von 50 ml wieder entfernt. Die Holzdübel und der Restbestand an Wasser wurden dann in Gläsern bei 121 °C für 20 min autoklaviert.

Aus den Petrischalen wurden Pilzmyzelplaques von 0,5 cm Größe herausgeschnitten und unter einer Sterilbank auf die sterilisierten Holzstücke aufgebracht.

Die Gläser hatten einen schraubbaren Verschlussdeckel aus Metall. In den Deckel war ein Loch gebohrt worden und mit einem autoklavierbaren Spritzen-Sterilfilteroder Spritzenvorsatzfilter (Luer Lock) versehen worden, der mit Hochtemperatur-Silikon befestigt wurde. Solch ein Spritzenvorsatzfilter ist in Figur 2 gezeigt. Das Ventil auf dem Deckel war erforderlich, um bei Bedarf Wasser nachfüllen zu können. Die Feuchtigkeit in den Gläsern wurde wöchentlich durch Inaugenscheinnahme kontrolliert und bei Bedarf Wasser nachgefüllt. Beim Durchwachsen in den Flaschen sind keine aktive Belüftung und daher eine hohe CO₂- Konzentration vorhanden.

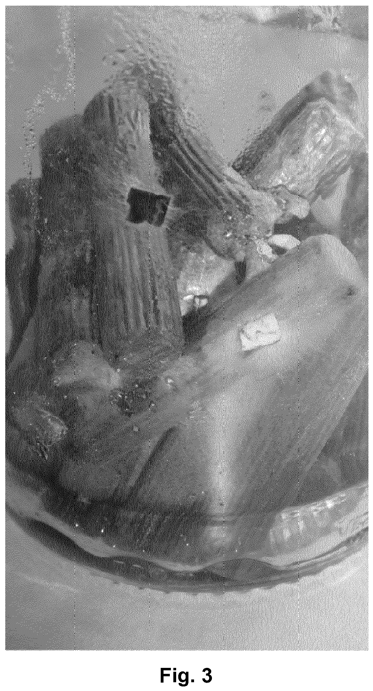

Im Verlaufe von 4-12 Wochen konnte ein Durchwachsen der Holzstücke mit blau-grünem Pilzmyzel beobachtet werden. Das Myzel wurde in dieser Zeit durch Schütteln der Behältnisse etwas im Glasgefäß verteilt. Solch eine blau-grünes Myzel des Pilzes *Chlorociboria sp.* aufgebracht auf Holzstücken in Glasgefäß ist in Figur 3 gezeigt.

Diese "durchwachsenen Holzdübel" wurden anschließend mittels vorher vorgenommener Bohrungen in größere Naturholzstücke eingeschoben. Diese Naturholzstücke wurden vorher bei 121 °C für 45 min autoklaviert.

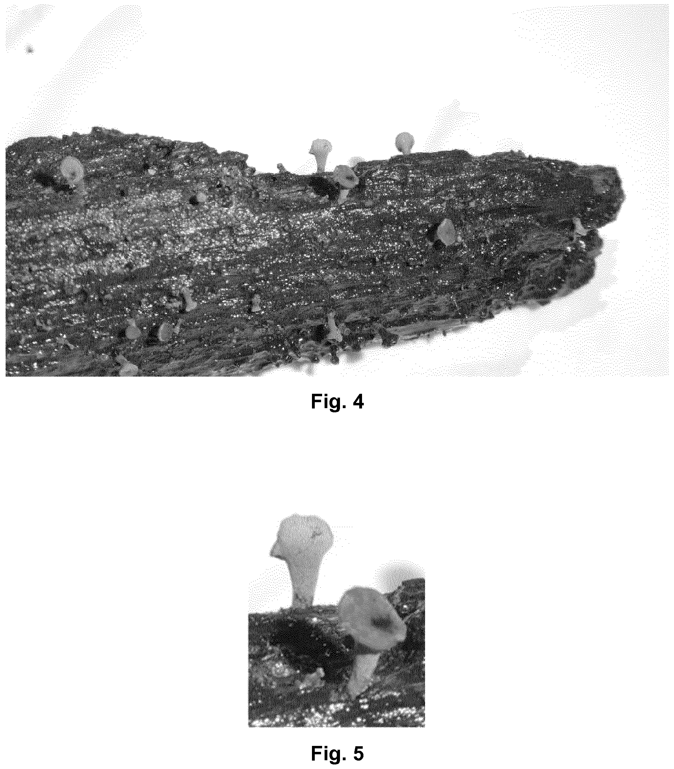

Diese Holstücke wurden anschließend bei einer Temperatur von bis zu 20 °C in einem Lagerraum ohne besondere Ausstattung in geeigneten handhabbaren Gefäßen eingelagert. Das Durchwachsen der Holzstücke erfolgte ohne besonderes Zutun, siehe auch Figuren 4 und 5.

Das Fruchten erfolgt spontan oder in der Klima Kammer nach 7 bis 21 Tagen bei maximaler Luftfeuchte, z.B. 85%, bei aktiver Belüftung und demnach einer möglichst hohen O₂ Sättigung. Beim Durchwachsen in Flaschen ist keine aktive Belüftung und daher eine hohe CO₂ Konzentration vorhanden.

Die hohe Luftfeuchte und die aktive Belüftung stellen naturgemäß einen Zielkonflikt dar, da durch die Belüftung die angefeuchtete Luft ausgetragen wird und bei weniger Belüftung aber mehr CO₂ vorhanden ist.

### Schritt (b): Ernten der Fruchtkörper

Die gewachsenen Fruchtkörper an den Holzstücken wurden vorsichtig mit Pinzetten abgebrochen. So ist sichergestellt, dass keine Holzbestandteile mit abgelesen werden und später das gewonnene Xylindein mit weiteren Inhaltsstoffen belastet wird. Die Fruchtkörper wurden in geeigneten verschließbaren Glasgefäßen gesammelt.

### Schritt (c): Einbringen der geernteten Fruchtkörper in ein Lösungsmittel

Die geernteten Fruchtkörper wurden entsprechend einer bevorzugten Ausführungsform zunächst mittels Mörser zerkleinert, in einen teebeutelartigen Filter eingeschlossen und in einem verschließbaren Glasgefäß in ein Lösungsmittel eingebracht, hier wurde das bevorzugte Lösungsmittel Aceton verwendet.

### Schritt (d): Extrahieren des Xylindeins aus den Fruchtkörpern mit dem Lösungsmittel

Über einen Zeitraum von 2-24 h wurde das Xylindein aus den Fruchtkörpern mit Aceton extrahiert, es entstand ein blau-grünes Gemisch. Je nach Wassergehalt und Xylindeingehalt in den Fruchtkörpern wurden diese mehrmals mittels Lösungsmittel behandelt, um das Xylindein erschöpfend zu extrahieren.

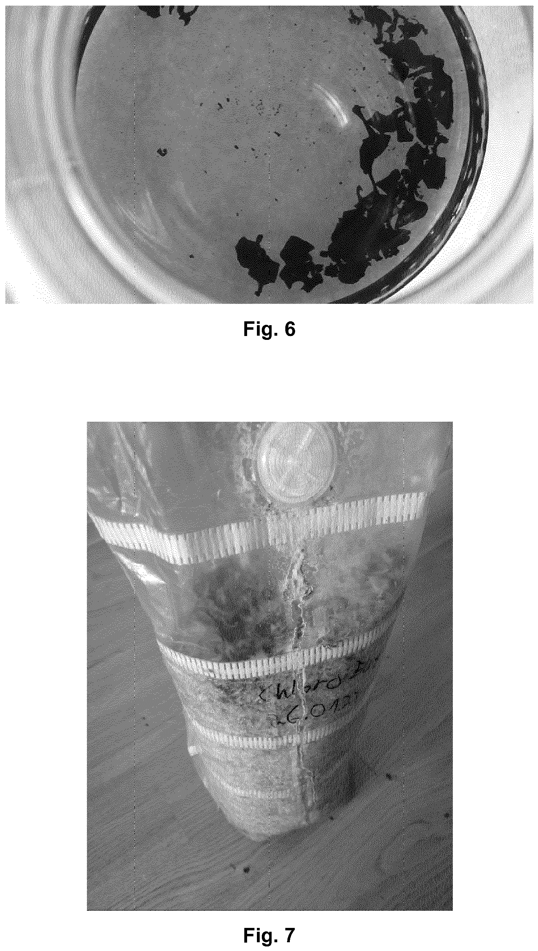

Solche Fruchtkörper in Aceton (ohne den zur Extraktion verwendeten "Teebeutel", um die Fruchtkörper zu sehen) sind in Figur 6 gezeigt.

### Schritt (e): Abtrennung des Lösungsmittels

Das entstandene Gemisch wurde mittels Papier-, Mikrofaser- oder Glasfaser filtriert, um die nicht lösbaren Rückstande der Fruchtkörper zurückzuhalten. Die Zugabe von Wasser bewirkte das Ausfällen des Xylindeins, weil die Löslichkeitsgrenze des Xylindeins im Wasser-Aceton-Gemisch unterschritten wurde. Gleichzeitig verbleiben wasserlösliche Verunreinigungen im Wasser-Aceton-Gemisch, während das Xylindein nach unten ausfällt. Anschließend muss der Überstand entfernt werden. Dies erfolgt durch Dekantieren des Überstandes, Abpipettieren der unteren Phase oder durch Einsatz eines Scheidentrichters, durch den die untere Phase, hier das Xylindein, abgelassen wird.

### Schritt (f): Trocknung des Xylindeins

Die gewonnenen Xylindeinpigmente wurden anschließend vorsichtig in adäquaten Schalen (z.B. Kristallisationsschalen) getrocknet. Temperatur und Dauer sind abhängig von dem gewünschten Gehalt an Restfeuchtigkeit. Zur vollständigen Trocknung 24 h bei 110 °C in einem Wärmeschrank.

### Beispiel 2

Das Verfahren erfolgte wie in Beispiel 1 jedoch unter Änderung der Vorgehensweise in Schritt (a).

### Schritt (a): Aufbringen und Kultivieren einer Chlorocibora sp. Pilzkultur auf sterilisiertem Naturholz

Das Pilzmyzel in den Petrischalen wurde in 100 ml sterilisiertes Leitungswasser überführt und mittels eines Zellendispensionsgerätes zerkleinert (püriert) und unter einer Sterilbank auf die sterilisierten Holzstücke aufgebracht.

### Beispiel 3

Das Verfahren erfolgte wie in Beispiel 1 jedoch unter Änderung der Vorgehensweise in Schritt (a).

### Schritt (a): Aufbringen und Kultivieren einer Chlorocibora sp. Pilzkultur auf sterilisiertem Naturholz

Anstelle von Holzstücken in der Größe von Holzdübeln wird gehäckseltes Holzgemisch verwandt. Solch eine blau-grünes Myzel des Pilzes *Chlorociboria sp.* aufgebracht auf gehäckseltem Holzgemisch ist in Figur 7 gezeigt.

### Beispiel 4

Das Verfahren erfolgte wie in Beispiel 1 jedoch nur bis Schritt (d).

### Schritte (e) und (f): Trocknung des Xylindeins

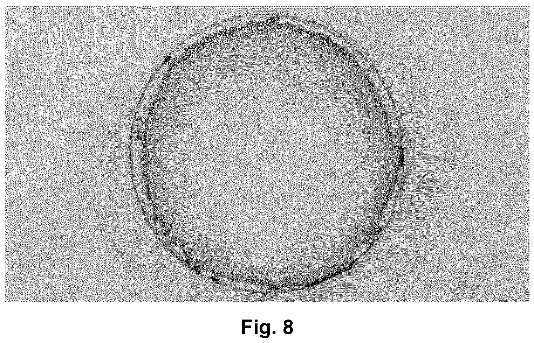

Alternativ zum Ausführungsbeispiel 1 wurden kristalline Xylindeinpigmente durch Evaporation des Wasser-Acetons-Gemischs (gegebenenfalls beschleunigt durch Wärmezufuhr) in Kristallisationsschalen gewonnen. Kristalline Xylindeinpigmente sind am äußeren Rand von Figur 8 zu erkennen.

## Patentansprüche

1. Verfahren zur biotechnologischen Gewinnung des blaugrünen Pilzpigments Xylindein aus Pilzfruchtkörpern, umfassend die folgenden Schritte
(a) Aufbringen, Kultivieren und zum Fruchten bringen einer *Chlorociboria sp.* Pilzkultur auf einem Nährmedium umfassend Naturholz oder Mischungen von verschiedenen Naturhölzern;
(b) Ernten der Fruchtkörper von der gemäß Schritt (a) kultivierten Pilzkultur;
(c) Einbringen der in Schritt (b) geernteten Fruchtkörper in ein Lösungsmittel oder Lösungsmittelgemisch und
(d) Extrahieren des Xylindeins aus den geernteten Fruchtkörpern mit dem Lösungsmittel oder Lösungsmittelgemisch.

2. Verfahren nach Anspruch 1, wobei in Schritt (a) das Nährmedium
(i) im Wesentlichen aus Naturholz oder aus Mischungen von verschiedenen Naturhölzern besteht;
(ii) vollständig sterilisierte, teilweise sterilisierte oder unbehandelte Naturhölzer umfasst; und/oder
(iii) Holzspäne, Holzdübel und/oder größere Holzstücke des Naturholzes/der Naturhölzer umfasst; und/oder
(iv) aus Weichhölzern und Harthölzern und vorzugsweise aus Weichhölzer wie Pappel, Weide und Birke besteht; und/oder
(v) zusätzliche organische oder anorganische Supplemente, insbesondere organischer Stickstoffverbindungen, umfasst,
wobei vorzugsweise das Nährmedium aus sterilisiertem Naturholz oder Mischungen desselben besteht.

3. Verfahren nach Anspruch 2, wobei das sterilisierte Naturholz durch Sterilisation des Naturholzes mittels Autoklave oder ionisierender Strahlung erhältlich ist.

4. Verfahren nach einem oder mehreren der Ansprüche 1 bis 3, wobei das Kultivieren der *Chlorociboria sp.* Pilzkultur in Schritt (a) unter partiell anaeroben Bedingungen in einem geschlossenen, aber nicht luftdichten Kultivierungsbehälter erfolgt, um vollständig anaerobe Bedingungen zu vermeiden, vorzugsweise bei erhöhtem CO₂ Gehalt, bei Temperaturen von 20 bis 30 °C und einem Feuchtigkeitsgrad, der in Richtung Maximalwert tendieren kann, erfolgt, und das zum Fruchten bringen der *Chlorociboria sp.* Pilzkultur durch Erhöhen des Sauerstoffgehalts in dem Kultivierungsbehälter relativ zu den Bedingungen der Kultivierung erfolgt.

5. Verfahren nach einem oder mehreren der Ansprüche 1 bis 4, weiterhin umfassend einen oder mehrere der folgenden Schritte
(ao) Vorkultivieren einer isolierten *Chlorociboria sp.* Pilzkultur oder Sporen derselben auf einem Agar-Nährmedium;
(e) Abtrennen des Xylindeins von dem Lösungsmittel oder Lösungsmittelgemisch;
(f) Trocknen des Xylindeins; und
(g) Reinigen des in Schritt (e) oder (f) erhaltenen Xylindeins.

6. Verfahren nach einem oder mehreren der Ansprüche 1 bis 5, wobei die die in Schritt (b) geernteten Fruchtkörper
(i) zunächst getrocknet und dann gemäß Schritt (c) in ein Lösungsmittel oder Lösungsmittelgemisch eingebracht werden, woraus nachfolgend das Pilzpigment Xylindein gemäß Schritt (d) extrahiert wird; oder
(ii) zunächst getrocknet, dann zerkleinert und dann gemäß Schritt (c) in ein Lösungsmittel oder Lösungsmittelgemisch eingebracht werden, woraus nachfolgend das Pilzpigment Xylindein gemäß Schritt (d) extrahiert wird; oder
(iii) zunächst zerkleinert, dann getrocknet und dann gemäß Schritt (c) in ein Lösungsmittel oder Lösungsmittelgemisch einbracht werden, woraus nachfolgend das Pilzpigment Xylindein gemäß Schritt (d) extrahiert wird; oder
(iv) zunächst zerkleinert und dann gemäß Schritt (c) in ein Lösungsmittel oder Lösungsmittelgemisch eingebracht werden, woraus nachfolgend das Pilzpigment Xylindein gemäß Schritt (d) mittels einer Flüssig-Flüssig-Extraktion extrahiert wird.

7. Verfahren nach einem oder mehreren der Ansprüche 1 bis 6, wobei die Pilzkultur *Chlorociboria sp.* ausgewählt ist aus *Chlorociboria aeruginascens* und *Chlorociboria aeruginosa.*

8. Verfahren nach einem der Ansprüche 1 bis 7, wobei das Lösungsmittel oder Lösungsmittelgemisch von Schritt (d) ausgewählt ist aus Dichlormethan, Ethanol, Chloroform, 2-Butanon, Aceton, Acetonitril, und Tetrahydrofuran, vorzugsweise Ethanol, 2-Butanon, Aceton, Acetonitril und Tetrahydrofuran umfasst, wobei Aceton und Aceton-Gemische wie Aceton/2-Butanon besonders bevorzugt sind.

9. Verfahren nach einem oder mehreren der Ansprüche 5 bis 8, wobei der Reinigungsschritt (g) vor oder nach dem Trocknungsschritt (f) erfolgt und vorzugsweise Umkristallisation und/oder Ausfällen umfasst.

10. Verfahren nach einem oder mehreren der Ansprüche 4 bis 8, wobei die in Schritt (a) eingesetzte *Chlorociboria sp.* Pilzkultur mindestens einem Vorkultivierungsschritt (ao) unterworfen werden.

11. Verfahren zur biotechnologischen Gewinnung des blaugrünen Pilzpigments Xylindein aus Pilzfruchtkörpern von *Chlorociboria sp,* umfassend Ernten von natürlich vorkommenden Fruchtkörpern von *Chlorociboria sp.* und Isolieren des Xylindeins daraus nach Schritt (c) und fortfolgenden Schritten gemäß einem oder mehreren der Ansprüchen 1 und 5 bis 9.

12. Verwendung des nach einem der Ansprüche 1 bis 11 gewonnen Xylindeins als Farbstoff für Farben, Lacke, Hautfarbstoffe (Tattoos), als Fluoreszenz-Farbstoff für medizinische Zwecke, als organisatorischer Halbleiter sowie für Vorprodukte dieser Anwendungen.

13. Verwendung nach Anspruch 12, wobei das nach Ansprüchen 1 bis 11 gewonnene, getrocknete Xylindein in Öl oder einem anderen Trägerstoff resuspendiert und als Anstrichfarbe und für Tattoos eingesetzt wird.
